# EUROPEAN PATENT SPECIFICATION

(11) **EP 1 373 541 B1**
(45) Date of publication and mention of the grant of the patent: **13.08.2008**
(21) Application number: 02759768.1
(22) Date of filing: 06.03.2002
(51) Int. Cl.: C12P 13/08, C12P 13/04

(54) **PROCESS FOR THE PRODUCTION OF L-AMINO ACIDS USING STRAINS OF THE FAMILY ENTEROBACTERIACEAE IN WHICH THE FRUR GENE IS DELETED**
VERFAHREN ZUR HERSTELLUNG VON L-AMINOSÄUREN MIT STÄMMEN DER FAMILIE ENTEROBACTERIACEAE IN DEN DAS FRUR-GEN DELETIERT WORDEN IST
PROCEDE DE PRODUCTION D'ACIDES AMINES L A L'AIDE DE SOUCHES DE LA FAMILLE DES ENTEROBACTERIACEAE DANS LESQUELLES LE GENE FRUR EST ELIMINE

(30) Priority: 03.04.2001 DE 10116518
(43) Date of publication of application: 02.01.2004
(73) Proprietor: Evonik Degussa GmbH, 45128 Essen (DE)
(72) Inventor: RIEPING, Mechthild, 33619 Bielefeld (DE); HERMANN, Thomas, 33739 Bielefeld (DE)
(86) International application number: PCT/EP2002/002420
(87) International publication number: WO 2002/081698

(56) References cited:
- EP-A- 0 237 819
- EP-A- 0 643 135
- EP-A- 0 952 221
- EP-A- 0 955 368
- WO-A-99/53035
- WO-A-02/081721
- WO-A-02/081722
- US-A- 4 278 765
- RAMSEIER T M: "Cra and the control of carbon flux via metabolic pathways." RESEARCH IN MICROBIOLOGY, vol. 147, no. 6-7, July 1996 (1996-07), pages 489-493, XP002241023 ISSN: 0923-2508
- JAHREIS K ET AL.: "Nucleotide sequence of the ilvH-fruR gene region of Escherichia coli K12 and Salmonella typhimurium LT2" MOLECULAR AND GENERAL GENETICS, vol. 226, no. 1/2, April 1991 (1991-04), pages 332-336, XP002937889 ISSN: 0026-8925 cited in the application
- MICHAL G: "Biochemical pathways: an atlas of biochemistry and molecular biology" 1999 , JOHN WILEY & SONS INC. AND SPEKTRUM AKADEMISCHER VERLAG , NEW YORK - HEIDELBERG XP002240819 ISBN: 0-471-33130-9 figures 3.8-1 and 3.8-2 figures 4.2-1, 4.5-1 and 4.5-2 paragraph [4.5.3]
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- DATABASE WPI Section Ch, Week 199148 Derwent Publications Ltd., London, GB; Class B05, AN 1991-351136 XP002241222 & JP 03 236786 A (KYOWA HAKKO KOGYO KK), 22 October 1991 (1991-10-22)
- JETTEN M S M ET AL.: "Recent advances in the physiology and genetics of amino acid-producing bacteria." CRC CRITICAL REVIEWS IN BIOTECHNOLOGY, vol. 15, no. 1, 1995, pages 73-103, XP000613291 ISSN: 0738-8551
- SAWERS G: "The anaerobic degradation of L-serine and L-threonine in enterobacteria: networks of pathways and regulatory signals" ARCHIVES OF MICROBIOLOGY, vol. 171, no. 1, 1998, pages 1-5, XP002953871 ISSN: 0302-8933
- BLEDIG A. ET AL: 'FruR mediates catabolite activation of pyruvate kinase (pykF) gene expression in Escherichia coli' JOURNAL OF BACTERIOLOGY vol. 178, no. 1, January 1996, pages 280 - 283

## Description

### Field of the Invention

The present invention relates to a process for the enzymatic production of the L-amino acids L-threonine, L-Lysing and L-valine using strains of the family Enterobacteriaceae in which the fruR gene is attenuated.

### Prior Art

L-amino acids, in particular L-threonine, are used in human medicine and in the pharmaceutical industry, in the foodstuffs industry, and most especially in animal nutrition.

It is known to produce L-amino acids by fermentation of strains of Enterobacteriaceae, in particular Escherichia coli (E. coli) and Serratia marcescens. On account of their great importance efforts are constantly being made to improve processes for producing the latter. Process improvements may relate to fermentation technology measures, such as for example stirring and provision of oxygen, or the composition of the nutrient media, such as for example the sugar concentration during the fermentation, or the working-up to the product form, for example by ion exchange chromatography, or the intrinsic performance properties of the microorganism itself.

Methods comprising mutagenesis, selection and mutant choice are employed in order to improve the performance properties of these microorganisms. In this way strains are obtained that are resistant to antimetabolites, such as for example the threonine analogue α-amino-β-hydroxyvaleric acid (AHV) or are auxotrophic for regulatorily important metabolites, and that produce L-amino acids such as for example L-threonine.

Methods of recombinant DNA technology have also been used for some years in order to improve strains of the family Enterobacteriaceae producing L-amino acids, by amplifying individual amino acid biosynthesis genes and investigating their effect on production.

### Object of the Invention

The object of the invention is to provide new measures for the improved enzymatic production of L-amino acids, in particular L-threonine.

### Summary of the Invention

The invention provides a process for the enzymatic production of L-amino acids, chosen from the group L-threonine, L-lysing and L-valine using microorganisms of the family Enterobacteriaceae that in particular already produce the desired L-amino acid and in which the nucleotide sequence coding for the fruR gene is attenuated.

### Detailed Description of the Invention

Where L-amino acids or amino acids are mentioned hereinafter, this is understood to mean one or more amino acids including their salts, selected from the group comprising L-threonine, L-valine and L-lysine and L⁻¹. L-threonine is particularly preferred.

The term "attenuation" describes in this connection the reduction or switching off of the intracellular activity of one or more enzymes (proteins) in a microorganism that are coded by the corresponding DNA, by using for example a weak promoter or a gene or allele that codes for a corresponding enzyme with a low activity and/or that inactivates the corresponding enzyme (protein) or gene, and optionally combining these measures.

By means of these attenuation measures the activity or concentration of the corresponding protein is generally reduced to 0 to 75%, 0 to 50%, 0 to 25%, 0 to 10% or 0 to 5% of the activity or concentration of the wild type protein, or the activity or concentration of the protein in the initial microorganism.

The process is characterized in that the following steps are carried out :
a) fermentation of microorganisms of the family Enterobacteriaceae producing the desired L-amino acid, in which the expression of the fruR gene, coding for the fructose repressor FruR, or of the nucleotide sequences coding for the gene product of the fruR gene is switched off,
b) enrichment of the corresponding L-amino acid in the medium or in the cells of the microorganisms of the family Enterobacteriaceae, and
c) isolation of the desired L-amino acid, in which optionally constituents of the fermentation broth and/or the biomass in its entirety or parts thereof remain in the product.

The microorganisms that are the subject of the present invention can produce L-amino acids from glucose, sucrose, lactose, fructose, maltose, molasses, optionally starch, optionally cellulose or from glycerol and ethanol. The microorganisms are members of the family Enterobacteriaceae selected from the genera Escherichia, Erwinia, Providencia and Serratia. The genera Escherichia and Serratia are preferred. In the case of the genus Escherichia the species Escherichia coli may in particular be mentioned, and in the case of the genus Serratia the species Serratia marcescens may in particular be mentioned.

Suitable strains of the genus Escherichia, in particular those of the species Escherichia coli, that produce in particular L-threonine, include for example:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Suitable strains of the genus Serratia, in particular of the species Serratia marcescens, that produce L-threonine include for example:
Serratia marcescens HNr21
Serratia marcescens TLr156
Serratia marcescens T2000

Strains of the family of Enterobacteriaceae producing L-threonine preferably have, *inter alia,* one or more of the genetic or phenotype features selected from the following group: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues such as for example valine hydroxamate, resistance to purine analogues such as for example 6-dimethylaminopurine, need for L-methionine, optionally partial and compensatable need for L-isoleucine, need for meso-diaininopimelic acid, auxotrophy with regard to threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally ability to utilise sucrose, enhancement of the threonine operon, enhancement of homoserine dehydrogenase, I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenol pyruvate carboxylase, optionally of the feedback-resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has now been found that microorganisms of the family Enterobacteriaceae after attenuation, in particular after switching off the fruR gene, produce L-amino acids, in particular L-threonine, in an improved way.

The nucleotide sequences of the Escherichia coli genes belong to the prior art and may also be obtained from the genome sequence of Escherichia coli published by Blattner et al. (Science 277, 1453 - 1462 (1997)).

The fruR gene is described inter alia by the following data:
Designation: Fructose repressor
EC-No.:
Reference: Jahreis et al., Molecular and General Genetics 226, 332-336 (1991)
Accession No.: AE000118

Ramseier T. M., Research in Microbiology, 147(6-7): 489-493, July 1996 generally discloses Cra-mediated control of key operons in carbon metabolism (Cra=FruR).

Bledig et al., J. Bacteriology 178(1): 280-283, January 1996 only discloses an E. coli strain lacking FruR.

### Comment: The fruR gene is also designated in the prior art as cra gene.

Apart from the described fruR gene, alleles of the gene may be used that result from the degeneracy of the genetic code or from functionally neutral sense mutations, the activity of the protein not being substantially altered.

In order to achieve an attenuation the expression of the gene or the catalytic properties of the enzyme proteins may for example be reduced or switched off. Optionally both measures may be combined.

The gene expression may be reduced by suitable culture conditions, by genetic alteration (mutation) of the signal structures of the gene expression, or also by antisense-RNA techniques. Signal structures of the gene expression are for example repressor genes, activator genes, operators, promoters, attenuators, ribosome-binding sites, the start codon and terminators. The person skilled in the art may find relevant information in, inter alia, articles by Jensen and Hammer (Biotechnology and Bioengineering 58: 191-195 (1998)), by Carrier and Keasling (Biotechnology Progress 15, 58-64 (1999)), Franch and Gerdes (Current Opinion in Microbiology 3, 159-164 (2000)) and in known textbooks of genetics and molecular biology, such as for example the textbook by Knippers ("Molekulare Genetik", 6th Edition, Georg Thieme Verlag, Stuttgart, Germany, 1995) or that by Winnacker ("Gene und Klone", VCH Verlagsgesellschaft, Weinheim, Germany, 1990).

Mutations that lead to a change or reduction of the catalytic properties of enzyme proteins are known from the prior art. As examples there may be mentioned the works by Qiu and Goodman (Journal of Biological Chemistry 272: 8611-8617 (1997)), Yano et al. (Proceedings of the National Academy of Sciences, USA 95, 5511-5515 (1998)), Wente and Schachmann (Journal of Biological Chemistry 266, 20833-20839 (1991)). Descriptive overviews may be obtained from known textbooks on genetics and molecular biology, such as for example that by Hagemann ("Allgemeine Genetik", Gustav Fischer Verlag, Stuttgart, 1986).

Suitable mutations include transitions, transversions, insertions and deletions. Depending on the action of the amino acid exchange on the enzyme activity, one speaks of missense mutations or nonsense mutations. Insertions or deletions of at least one base pair in a gene lead to frame shift mutations, which in turn lead to the incorporation of false amino acids or the premature termination of a translation. If as a result of the mutation a stop codon is formed in the coding region, this also leads to a premature termination of the translation. Deletions of several codons typically lead to a complete disruption of the enzyme activity. Details regarding the production of such mutations belong to the prior art and may be obtained from known textbooks on genetics and molecular biology, such as for example the textbook by Knippers ("Molekulare Genetik", 6th Edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

Suitable mutations in the genes such as for example deletion mutations may be incorporated by gene and/or allele exchange in suitable strains.

A conventional method is the method of gene exchange by means of a conditionally replicating pSC101 derivate pMAK705 described by Hamilton et al. (Journal of Bacteriology 171, 4617 - 4622 (1989)). Other methods described in the prior art, such as for example that of Martinez-Morales et al. (Journal of Bacteriology 181, 7143-7148 (1999)) or that of Boyd et al. (Journal of Bacteriology 182, 842-847 (2000)) may likewise be used.

It is also possible to transfer mutations in the respective genes or mutations relating to the expression of the relevant genes, by conjugation or transduction into various strains.

Furthermore for the production of L-threonine, using strains of the family Enterobacteriaceae it may be advantageous in addition to the attenuation of the fruR gene also to enhance one or more enzymes of the known threonine biosynthesis pathway or enzymes of anaplerotic metabolism or enzymes for the production of reduced nicotinaminde-adenine-dinucleotide phosphate.

The term "enhancement" describes in this connection the raising of the intracellular activity of one or more enzymes or proteins in a microorganism that are coded by the corresponding DNA, by for example increasing the number of copies of the gene or genes, using a strong promoter or a gene that codes for a corresponding enzyme or protein having a high activity, and optionally by combining these measures.

By means of the aforementioned enhancement measures, in particular overexpression, the activity or concentration of the corresponding protein is in general raised by at least 10%, 25%, 50%, 75%, 100%, 150%, 200%, 300%, 400% or 500%, at most up to 1000% or 2000% referred to that of the wild type protein and/or the activity or concentration of the protein in the initial microorganism.

Thus, one or more of the genes selected from the following group may for example be simultaneously overexpressed:
• the thrABC operon coding for aspartate kinase, homoserine dehydrogenase, homoserine kinase and threonine synthase (US-A-4,278,765),
• the pyc gene coding for pyruvate carboxylase (DE-A-19 831 609),
• the pps gene coding for phosphoenol pyruvate synthase (Molecular and General Genetics 231:332 (1992)),
• the ppc gene coding for phosphoenol pyruvate carboxylase (Gene 31:279-283 (1984)),
• the genes pntA and pntB coding for transhydrogenase (European Journal of Biochemistry 158:647-653 (1986)),
• the gene rhtB imparting homoserine resistance (EP-A-0 994 190),
• the mqo gene coding for malate:quinone oxidoreductase (DE 100 348 33.5),
• the gene rhtC imparting threonine resistance (EP-A-1 013 765), and
• the thrE gene of Corynebacterium glutamicum coding for threonine export (DE 100 264 94.8).

The use of endogenous genes is in general preferred. The term "endogenous genes" or "endogenous nucleotide sequences" is understood to mean the genes or nucleotide sequences present in the population of a species.

Furthermore for the production of L-threonine, it may be advantageous in addition to the attenuation of the fruR gene also to attenuate, in particular to switch off or reduce the expression of one or more of the genes selected from the following group:
• the tdh gene coding for threonine dehydrogenase (Ravnikar and Somerville, Journal of Bacteriology 169, 4716-4721 (1987)),
• the mdh gene coding for malate dehydrogenase (E.C. 1.1.1.37) (Vogel et al., Archives in Microbiology 149, 36-42 (1987)),
• the gene product of the open reading frame (orf) yjfA (Accession Number AAC77180 of the National Center for Biotechnology Information (NCBI, Bethesda, MD, USA)),
• the gene product of the open reading frame (orf) ytfP (Accession Number AAC77179 of the National Center for Biotechnology Information (NCBI, Bethesda, MD, USA)),
• the pckA gene coding for the enzyme phosphoenol pyruvate carboxykinase (Medina et al. (Journal of Bacteriology 172, 7151-7156 (1990)),
• the poxB gene coding for pyruvate oxidase (Grabau and Cronan (Nucleic Acids Research 14 (13), 5449-5460 (1986)),
• the aceA gene coding for isocitrate lyase (EC-No.: 4.1.3.1) (Matsuoko and McFadden; Journal of Bacteriology 170, 4528-4536 (1988) and Accession No.: AE000474), and
• the dgsA gene coding for the regulator of the phosphotransferase system (Hosono et al., Bioscience, Biotechnology and Biochemistry 59, 256-261 (1995) and Accession No.: AE000255)

Furthermore for the production of L-amino acids, in particular L-threonine, it may be advantageous in addition to the attenuation of the fruR gene also to switch off undesirable secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention may be cultivated in a batch process (batch cultivation), in a fed batch process (feed process) or in a repeated fed batch process (repetitive feed process). A summary of known cultivation methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Brunswick/Wiesbaden, 1994)).

The culture medium to be used must appropriately satisfy the requirements of the respective strains. Descriptions of culture media of various microorganisms are contained in the handbook "Manual of Methods for General Bacteriology" of the American Society for Bacteriology (Washington D.C., USA, 1981).

As carbon sources, sugars and carbohydrates such as for example glucose, sucrose, lactose, fructose, maltose, molasses, starch and optionally cellulose, oils and fats such as for example soya bean oil, sunflower oil, groundnut oil and coconut oil, fatty acids such as for example palmitic acid, stearic acid and linoleic acid, alcohols such as for example glycerol and ethanol, and organic acids such as for example acetic acid, may be used. These substances may be used individually or as a mixture.

As nitrogen source, organic nitrogen-containing compounds such as peptones, yeast extract, meat extract, malt extract, maize steep liquor, soya bean flour and urea or inorganic compounds such as ammonium sulfate, ammonium chloride, ammonium phosphate, ammonium carbonate and ammonium nitrate may be used. The nitrogen sources may be used individually or as a mixture.

As phosphorus source, phosphoric acid, potassium dihydrogen phosphate or dipotassium hydrogen phosphate or the corresponding sodium-containing salts may be used. The culture medium must furthermore contain salts of metals, such as for example magnesium sulfate or iron sulfate, that are necessary for growth. Finally, essential growth promoters such as amino acids and vitamins may be used in addition to the aforementioned substances. Apart from these, suitable precursors may be added to the culture medium. The aforementioned starting substances may be added to the culture in the form of a single batch or may be metered in in an appropriate manner during the cultivation.

In order to regulate the pH of the culture basic compounds such as sodium hydroxide, potassium hydroxide, ammonia or ammonia water, or acidic compounds such as phosphoric acid or sulfuric acid are used as appropriate. In order to control foam formation antifoaming agents such as for example fatty acid polyglycol esters may be used. In order to maintain the stability of plasmids, suitable selectively acting substances, for example antibiotics, may be added to the medium. In order to maintain aerobic conditions, oxygen or oxygen-containing gas mixtures such as for example air are fed into the culture. The temperature of the culture is normally 25°C to 45°C, and preferably 30°C to 40°C. Cultivation is continued until a maximum amount of L-amino acids (or L-threonine) has been formed. This target is normally achieved within 10 hours to 160 hours.

The L-amino acids may be analyzed by anion exchange chromotography followed by ninhydrin derivation, as described by Spackman et al. (Analytical Chemistry, 30, (1958), 1190), or by reversed phase HPLC, as described by Lindroth et al. (Analytical Chemistry (1979) 51: 1167-1174).

The process according to the invention can be used for the enzymatic production of L-amino acids L-threonine, L-valine and L-lysine, in particular L-threonine. A pure culture of the Escherichia coli K-12 strain DH5α/pMAK705 was filed as DSM 13720 on 8 September 2000 at the German Collection for Microorganisms and Cell Cultures (DSMZ, Brunswick, Germany) according to the Budapest Convention.

The present invention is described in more detail hereinafter with the aid of examples of implementation.

The isolation of plasmid DNA from Escherichia coli as well as all techniques for the restriction, ligation, Klenow treatment and alkaline phosphatase treatment are carried out according to Sambrook et al. (Molecular Cloning - A Laboratory Manual (1989) Cold Spring Harbor Laboratory Press). The transformation of Escherichia coli is, unless otherwise described, carried out according to Chung et al. (Proceedings of the National Academy of Sciences of the United States of America, USA (1989) 86: 2172-2175).

The incubation temperature in the production of strains and transformants is 37°C. In the gene exchange process according to Hamilton et al, temperatures of 30°C and 44°C are used.

### Example 1

### Construction of the deletion mutation of the fruR gene

Parts of the gene regions and parts of the 5'- and 3'-region of the fruR gene from Escherichia coli K12 lying upstream and downstream of the fruR gene are amplified using the polymerase chain reaction (PCR) as well as synthetic oligonucleotides. Starting from the nucleotide sequence of the fruR gene and sequences in E. coli K12 MG1655 DNA (SEQ ID No. 1, Accession Number AE000118) lying upstream and downstream, the following PCR primers are synthesized (MWG Biotech, Ebersberg, Germany):
fruR'5'-1: 5' - ATGAATCAGGCGCGTTATCC - 3' (SEQ ID No. 3)
fruR'5'-2: 5' - TTGTCGCTCACACGGTATTG - 3' (SEQ ID No. 4)
fruR'3'-1: 5' - AGCGTGTGCTGGAGATTGTC - 3' (SEQ ID No. 5)
fruR'3'-2: 5' - AGCCAGTCACAAGGCATACC - 3' (SEQ ID No. 6)

The chromosomal E. coli K12 MG1655 DNA used for the PCR is isolated according to the manufacturer's, instructions using "Qiagen Genomic-tips 100/G" (QIAGEN, Hilden, Germany). A ca. 750 bp large DNA fragment from the 5' region of the fruR gene region (designated fruR1) and a ca. 650 bp large DNA fragment from the 3' region of the fruR gene region (designated as fruR2) may be amplified with the specific primers under standard PCR conditions (Innis et al. (1990) PCR Protocols. A Guide to Methods and Applications, Academic Press) with the taq-DNA-polymerase (Gibco-BRL, Eggenstein, Germany). The PCR products are ligated according to the manufacturer's instructions in each case with the vector pCR2.1TOPO (TOPO TA Cloning Kit, Invitrogen, Groningen, Netherlands) and transformed in the E. coli strain TOP10F'. The selection of plasmid-carrying cells is carried out on LB agar to which 50 µg/ml of ampicillin has been added. After the plasmid DNA isolation the vector pCR2.1TOPOfruR2 is cleaved with the restriction enzyme NotI and the supernatant 3'-ends are treated with Klenow enzyme. After the restriction with the enzyme SpeI the fruR2 fragment is separated in 0.8% agarose gel and isolated using the QIAquick Gel Extraction Kit (QIAGEN, Hilden, Germany). After the plasmid DNA isolation, the vector pCR2.1TOPOfruR1 is cleaved with the enzymes EcoRV and XbaI and ligated with the isolated fruR2 fragment. The E. coli strain DH5α is transformed with the ligation batch and plasmid-carrying cells are selected on LB agar to which 50 µg/ml of ampicillin has been added. After the plasmid DNA isolation those plasmids in which the mutagenic DNA sequence illustrated in SEQ ID No. 7 is present in cloned form are detected by control cleavage with the enzymes HindIII, EcoRV and PvuI. One of the plasmids is designated pCR2.1TOPOΔfruR.

### Example 2

### Construction of the exchange vector pMAK705ΔfruR

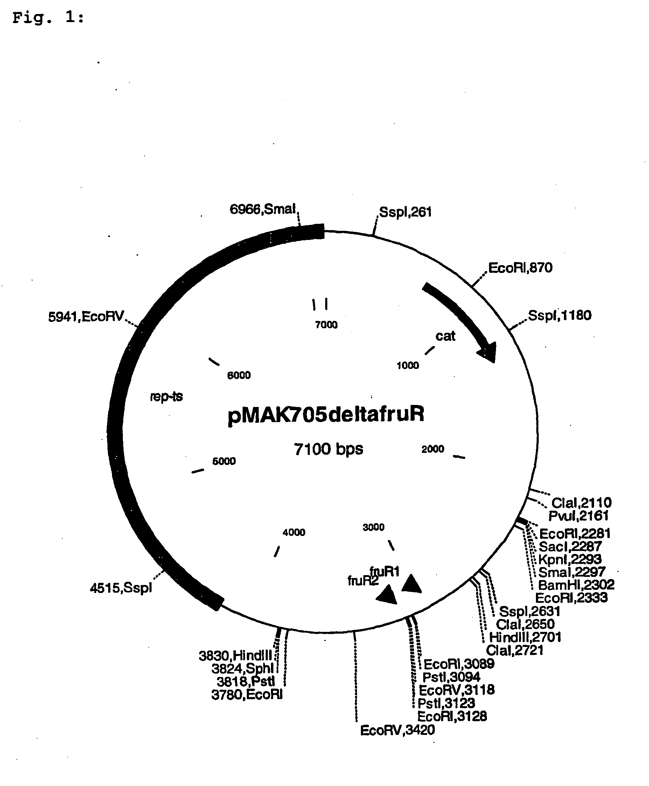

The fruR allele described in Example 1 is isolated from the vector pCR2.1TOPOΔfruR after restriction with the enzyme Eco0109I, treatment of the supernatant 3'-ends with Klenow enzyme, restriction with the enzyme BamHI and separation in 0.8% agarose gel, and ligated with the plasmid pMAK705 (Hamilton et al. (1989) Journal of Bacteriology 171, 4617 - 4622) that has been digested with the enzymes HincII and BamHI. The ligation batch is transformed in DH5α and plasmid-carrying cells are selected on LB agar to which 20 µg/ml chloramphenicol had been added. Successful cloning is detected after plasmid DNA isolation and cleavage with the enzymes HindIII, BamHI, EcoRV, ScaI and SpeI. The resultant exchange vector pMAK705ΔfruR (= pMAK705deltafruR) is shown in Fig. 1.

### Example 3

Site-specific mutagenesis of the fruR gene in the E. coli strain MG442

The E. coli strain MG442 producing L-threonine is described in patent specification US-A- 4,278,765 and is filed as CMIM B-1628 at the Russian National Collection for Industrial Microorganisms (VKPM, Moscow, Russia).

For the exchange of the chromosomal fruR gene by the plasmid-coded deletion construct, MG442 is transformed with the plasmid pMAK705ΔfruR. The gene exchange is carried out by the selection process described by Hamilton et al. (1989) Journal of Bacteriology 171, 4617 - 4622) and is verified by standard PCR methods (Innis et al. (1990) PCR Protocols. A guide to methods and applications, Academic Press) with the following oligonucleotide primers:
fruR'5'-1: 5' - ATGAATCAGGCGCGTTATCC - 3' (SEQ ID No. 3)
fruR'3'-2: 5' - AGCCAGTCACAAGGCATACC - 3' (SEQ ID No. 6)

After exchange has been carried out the form of the ΔfruR allele illustrated in SEQ ID No. 8 is present in MG442. The resultant strain is designated MG442ΔfruR.

### Example 4

Production of L-threonine using the strain MG442ΔfruR

MG442ΔfruR is cultivated on minimal medium having the following composition: 3.5 g/l Na₂HPO₄·2H₂O, 1.5 g/l KH₂PO₄, 1 g/l NH₄Cl, 0.1 g/l MgSO₄·7H₂O, 2 g/l glucose and 20 g/l agar. The formation of L-threonine is checked in batch cultures of 10 ml that are contained in 100 ml Erlenmeyer flasks. For this, 10 ml of preculture medium of the following composition: 2 g/l yeast extract, 10 g/l (NH₄)₂SO₄, 1 g/l KH₂PO₄, 0.5 g/l MgSO₄·7H₂O, 15 g/l CaCO₃, 20 g/l glucose are inoculated and incubated for 16 hours at 37°C and 180 rpm in an ESR incubator from Kühner AG (Birsfelden, Switzerland). 250 µl of this preculture are reinoculated in 10 ml of production medium (25 g/l (NH₄)₂SO₄, 2 g/l KH₂PO₄, 1 g/l MgSO₄·7H₂O, 0.03 g/l FeSO₄·7H₂O, 0.018 g/l MnSO₄·1H₂O, 30 g/l CaCO₃ and 20 g/l glucose) and incubated for 48 hours at 37°C. After incubation the optical density (OD) of the culture suspension is measured with an LP2W photometer from the Dr. Lange company (Dusseldorf, Germany) at a measurement wavelength of 660 nm.

The concentration of formed L-threonine is then determined in the sterile-filtered culture supernatant using an amino acid analyzer from Eppendorf-BioTronik (Hamburg, Germany)
• BglII: restriction endonuclease from Bacillus globigii
• ClaI: restriction endonuclease from Caryphanon latu
• EcoRI: restriction endonuclease from Escherichia col
• EcoRV: restriction endonuclease from Escherichia col
• HindIII: restriction endonuclease from Haemophilus influenzae
• KpnI: restriction endonuclease from Klebsiella pneumoniae
• PstI: restriction endonuclease from Providencia stuartii
• PvuI: restriction endonuclease from Proteus vulgari
• SacI: restriction endonuclease from Streptomyces achromogenes
• SalI: restriction endonuclease from Streptomyces albus
• SmaI: restriction endonuclease from Serratia marcescens
• SphI: restriction endonuclease from Streptomyces phaeochromogenes
• SspI: restriction endonuclease from Sphaerotilus species
• XbaI: restriction endonuclease from Xanthomonas badrii
• XhoI: restriction endonuclease from Xanthomonas holcicola

## Claims

1. Process for the production of L-amino acids, chosen from the group L-threonine, L-lysine and L-valine wherein the following steps are carried out:
a) fermentation of the microorganisms of the family Enterobacteriaceae producing the desired L-amino acid, in which the expression of the fruR gene, coding for the fructose repressor FruR, or of the nucleotide sequences coding for the gene product of the fruR gene is switched off,
b) enrichment of the L-amino acid in the medium or in the cells of the microorganisms, and
c) isolation of the L-amino acid, in which optionally constituents of the fermentation broth and/or the biomass in its entirety or portions thereof remain in the product.

2. Process according to claim 1, wherein for the production of L-threonine microorganisms of the family Enterobacteriaceae are fermented in which at the same time one or more of the genes selected from the following group is or are overexpressed:
2.1 the thrABC operon coding for aspartate kinase, homoserine dehydrogenase, homoserine kinase and threonine synthase,
2.2 the pyc gene coding for pyruvate carboxylase,
2.3 the pps gene coding for phosphoenol pyruvate synthase,
2.4 the ppc gene coding for phosphoenol pyruvate carboxylase,
2.5 the genes pntA and pntB coding for transhydrogenase,
2.6 the Escherichia coli rhtB gene coding for a protein imparting homoserine resistance,
2.7 the mqo gene coding for malate:quinone oxidoreductase,
2.8 the Escherichia coli rhtC gene coding for a protein imparting threonine resistance, and
2.9 the thrE gene coding for threonine export.

3. Process according to claim 1, wherein for the production of L-threonine microorganisms of the family Enterobacteriaceae are fermented in which at the same time the expression of one or more of the genes selected from the following group is switched off:
3.1 the tdh gene coding for threonine dehydrogenase,
3.2 the mdh gene coding for malate-dehydrogenase,
3.3 the gene product of the open reading frame (orf) yjfA of E. coli, when said microorganism is Escherichia coli,
3.4 the gene product of the open reading frame (orf) ytfP of E. coli, when said microorganism is Escherichia coli,
3.5 the pckA gene coding for phosphoenol pyruvate carboxykinase,
3.6 the poxB gene coding for pyruvate oxidase,
3.7 the aceA gene coding for isocitrate lyase,
3.8 the dgsA gene coding for the regulator of the phosphotransferase system.

4. L-threonine producing microorganisms of the species Escherichia coli, wherein at least the expression of the fruR gene, coding for the fructose repressor, and of the open reading frame (orf) yjfA, or the expression of nucleotide sequences which code for the fruR gene product and of the open reading frame yjfA is switched off.

## Patentansprüche

1. Verfahren zur Herstellung von L-Aminosäuren, ausgewählt aus der Gruppe L-Threonin, L-Lysin und L-Valin, bei dem man die folgenden Schritte durchführt:
a) Fermentation der Mikroorganismen der Familie Enterobacteriaceae, die die gewünschte .L-Aminosäure produzieren und in denen die Expression des fruR-Gens, das für den Fructoserepressor FruR codiert, oder der Nukleotidsequenzen, die für das Genproduckt des fruR-Gens codieren, ausgeschaltet ist,
b) Anreicherung der L-Aminosäure im Medium oder in den Zellen der Mikroorganismen und
c) Isolierung der L-Aminosäure, wobei gegebenenfalls Bestandteile der Fermentationsbrühe und/oder die Biomasse insgesamt oder teilweise im Produkt verbleiben.

2. Verfahren gemäß Anspruch 1, wobei man zur Herstellung von L-Threonin Mikroorganismen der Familie Enterobacteriaceae fermentiert, in denen gleichzeitig eines oder mehrere der Gene, ausgewählt aus der folgenden Gruppe, überexprimiert wird bzw. werden:
2.1 das für Aspartatkinase, Homoserin-Dehydrogenase, Homoserinkinase und Threonin-Synthase codierende thrABC-Operon,
2.2 das für die Pyruvat-Carboxylase codierende pyc-Gen,
2.3 das für die Phosphoenolpyruvat-Synthase codierende pps-Gen,
2.4 das für die Phosphoenolpyruvat-Carboxylase codierende ppc-Gen,
2.5 die für Transhydrogenase codierenden Gene pntA und pntB,
2.6 das für ein Homoserin-Resistenz verleihendes Protein codierende rhtB-Gen aus Escherichia coli,
2.7 das für die Malat:Chinon-Oxidoreduktase codierende mqo-Gen,
2.8 das für ein Threonin-Resistenz verleihendes Protein codierende rhtC-Gen aus Escherichia coli und
2.9 das für Threonin-Export codierende thrE-Gen.

3. Verfahren gemäß Anspruch 1, wobei man zur Herstellung von L-Threonin Mikroorganismen der Familie Enterobacteriaceae fermentiert, in denen gleichzeitig die Expression eines oder mehrerer Gene, ausgewählt aus der nachfolgenden Gruppe, ausgeschaltet ist:
3.1 das für die Threonin-Dehydrogenase codierende tdh-Gen,
3.2 das für die Malat-Dehydrogenase codierende mdh-Gen,
3.3 das Genprodukt des offenen Leserasters (orf) yjfA aus E. coli, wenn es sich bei dem Mikroorganismus um Escherichia coli handelt,
3.4 das Genprodukt des offenen Leserasters (orf) ytfP aus E. coli, wenn es sich bei dem Mikroorganismus um Escherichia coli handelt,
3.5 das für die Phosphoenolpyruvat-Carboxykinase codierende pckA-Gen,
3.6 das für die Pyruvat-Oxidase codierende poxB-Gen,
3.7 das für die Isocitrat-Lyase codierende aceA-Gen,
3.8 das für den Regulator des Phosphotransferase-Systems codierende dgsA-Gen.

4. L-Threonin produzierende Mikroorganismen der Spezies Escherichia coli, wobei wenigstens die Expression des für den Fructoserepressor codierenden fruR-Gens und des offenen Leserasters (orf) yjfA oder die Expression von Nukleotidsequenzen, die für das fruR-Genproduckt codieren, und des offenen Leserasters yjfA ausgeschaltet ist.

## Revendications

1. Procédé de production d'acides L-aminés, choisis dans le groupe comprenant la L-thréonine, la L-lysine et la L-valine, **caractérisé en ce que** les étapes suivantes sont effectuées :
a) la fermentation des microorganismes de la famille des Enterobacteriaceae produisant l'acide L-aminé désiré, dans lesquels l'expression du gène fruR, codant pour le répresseur de fructose fruR, ou des séquences nucléotidiques codant pour le produit génique du gène fruR, est éteinte,
b) l'enrichissement de l'acide L-aminé dans le milieu ou dans les cellules des microorganismes, et
c) l'isolement de l'acide L-aminé, où éventuellement les constituants du bouillon de fermentation et/ou la biomasse dans son intégralité ou des portions de celle-ci restent dans le produit.

2. Procédé selon la revendication 1, **caractérisé en ce que**, pour la production de la L-thréonine, des microorganismes de la famille des Enterobacteriaceae sont mis en fermentation, dans lesquels, en même temps, un ou plusieurs des gènes choisis dans le groupe suivant sont surexprimés :
2.1 l'opéron thrABC codant pour l'aspartate kinase, l'homosérine déshydrogénase, l'homosérine kinase et la thréonine synthase,
2.2 le gène pyc codant pour la pyruvate carboxylase,
2.3 le gène pps codant pour la phosphoénol pyruvate synthase,
2.4 le gène ppc codant pour la phosphoénol pyruvate carboxylase,
2.5 les gènes pntA et pntB codant pour la transhydrogénase,
2.6 le gène rhtB d'Escherichia coli codant pour une protéine donnant la résistance à l'homosérine,
2.7 le gène mqo codant pour la malate:quinone oxydoréductase,
2.8 le gène rhtC d'Escherichia coli codant pour une protéine donnant la résistance à la thréonine, et
2.9 le gène thrE codant pour l'exportation de la thréonine.

3. Procédé selon la revendication 1, **caractérisé en ce que**, pour la production de la L-thréonine, des microorganismes de la famille des Enterobacteriaceae sont mis en fermentation, dans lesquels, en même temps, l'expression d'un ou plusieurs des gènes choisis dans le groupe suivant est éteinte :
3.1 le gène tdh codant pour la thréonine déshydrogénase,
3.2 le gène mdh codant pour la malate déshydrogénase,
3.3 le produit du gène du cadre de lecture ouvert (orf) yjfA d'E. coli, lorsque ledit microorganisme est l'Escherichia coli,
3.4 le produit du gène du cadre de lecture ouvert (orf) ytfP d'E. coli, lorsque ledit microorganisme est l'Escherichia coli,
3.5 le gène pckA codant pour la phosphoénol pyruvate carboxykinase,
3.6 le gène poxB codant pour la pyruvate oxydase.
3.7 le gène aceA codant pour l'isocitrate lyase,
3.8 le gène dgsA codant pour le régulateur du système phosphotransférase.

4. Microorganismes producteurs de la L-thréonine de l'espèce Escherichia coli, **caractérisés en ce qu'**au moins l'expression du gène fruR, codant pour le répresseur de fructose, et du cadre de lecture ouvert (orf) yjfA, ou l'expression des séquences nucléotidiques codant pour le produit du gène fruR et du cadre de lecture ouvert yjfA, est éteinte.


### SEQUENCE LISTING

<110> Degussa AG
<120> Process for the production of L-amino acids using strains of the family Enterobacteriaceae containing an attenuated fruR gene
<130> 020005 BT
<160> 8
<170> PatentIn version 3.1
<210> 1
   <211> 2166
   <212> DNA
   <213> Escherichia coli
<220>
   <221> CDS
   <222> (641)..(1645)
   <223> fruR gene
   <400> 1
   atgaatcagg cgcgttatcc cgcgtgattg gccttttttc ccagcgtggc tacaacattg    60
   aaagcctgac cgttgcgcca accgacgatc cgacattatc gcgtatgacc atccagaccg   120
   tgggcgatga aaaagtactt gagcagatcg aaaagcaatt acacaaactg gtcgatgtct   180
   tgcgcgtgag tgagttgggg cagggcgcgc atgttgagcg ggaaatcatg ctggtgaaaa   240
   ttcaggccag cggttacggg cgtgacgaag tgaaacgtaa tacggaaata ttccgtgggc   300
   aaattatcga tgtcacaccc tcgctttata ccgttcaatt agcaggcacc agcggtaagc   360
   ttgatgcatt tttagcatcg attcgcgatg tggcgaaaat tgtggaggtt gctcgctctg   420
   gtgtggtcgg actttcgcgc ggcgataaaa taatgcgttg agaatgatct caatgcgcaa   480
   tttacagccc aacatgtcac gttgggcttt ttttgcgaaa tcagtgggaa cctggaataa   540
   aagcagttgc cgcagttaat tttctgcgct tagatgttaa tgaatttaac ccataccagt   600
   acaatggcta tggtttttac attttacgca aggggcaatt gtg aaa ctg gat gaa     655
                                               Met Lys Leu Asp Glu
                                               1               5
   atc gct cgg ctg gcg gga gtg tcg cgg acc act gca agc tat gtt att     703
   Ile Ala Arg Leu Ala Gly Val Ser Arg Thr Thr Ala Ser Tyr Val Ile
                   10                  15                  20
   aac ggc aaa gcg aag caa tac cgt gtg agc gac aaa acc gtt gaa aaa     751
   Asn Gly Lys Ala Lys Gln Tyr Arg Val Ser Asp Lys Thr Val Glu Lys
               25                  30                  35
   gtc atg gct gtg gtg cgt gag cac aat tac cac ccg aac gcc gtg gca     799
   Val Met Ala Val Val Arg Glu His Asn Tyr His Pro Asn Ala Val Ala
           40                  45                  50
   gct ggg ctt cgt gct gga cgc aca cgt tct att ggt ctt gtg atc ccc     847
   Ala Gly Leu Arg Ala Gly Arg Thr Arg Ser Ile Gly Leu Val Ile Pro
       55                  60                  65
   gat ctg gag aac acc agc tat acc cgc atc gct aac tat ctt gaa cgc      895
   Asp Leu Glu Asn Thr Ser Tyr Thr Arg Ile Ala Asn Tyr Leu Glu Arg
   70                  75                  80                  85
   cag gcg cgg caa cgg ggt tat caa ctg ctg att gcc tgc tca gaa gat      943
   Gln Ala Arg Gln Arg Gly Tyr Gln Leu Leu Ile Ala Cys Ser Glu Asp
                   90                  95                  100
   cag cca gac aac gaa atg cgg tgc att gag cac ctt tta cag cgt cag      991
   Gln Pro Asp Asn Glu Met Arg Cys Ile Glu His Leu Leu Gln Arg Gln
               105                 110                 115
   gtt gat gcc att att gtt tcg acg tcg ttg cct cct gag cat cct ttt     1039
   Val Asp Ala Ile Ile Val Ser Thr Ser Leu Pro Pro Glu His Pro Phe
           120                 125                 130
   tat caa cgc tgg gct aac gac ccg ttc ccg att gtc gcg ctg gac cgc     1087
   Tyr Gln Arg Trp Ala Asn Asp Pro Phe Pro Ile Val Ala Leu Asp Arg
       135                 140                 145
   gcc ctc gat cgt gaa cac ttc acc agc gtg gtt ggt gcc gat cag gat     1135
   Ala Leu Asp Arg Glu His Phe Thr Ser Val Val Gly Ala Asp Gln Asp
   150                 155                 160                 165
   gat gcc gaa atg ctg gcg gaa gag tta cgt aag ttt ccc gcc gag acg     1183
   Asp Ala Glu Met Leu Ala Glu Glu Leu Arg Lys Phe Pro Ala Glu Thr
                   170                 175                 180
   gtg ctt tat ctt ggt gcg cta ccg gag ctt tct gtc agc ttc ctg cgt     1231
   Val Leu Tyr Leu Gly Ala Leu Pro Glu Leu Ser Val Ser Phe Leu Arg
               185                 190                 195
   gaa caa ggt ttc cgt act gcc tgg aaa gat gat ccg cgc gaa gtg cat     1279
   Glu Gln Gly Phe Arg Thr Ala Trp Lys Asp Asp Pro Arg Glu Val His
           200                 205                 210
   ttc ctg tat gcc aac agc tat gag cgg gag gcg gct gcc cag tta ttc     1327
   Phe Leu Tyr Ala Asn Ser Tyr Glu Arg Glu Ala Ala Ala Gln Leu Phe
       215                 220                 225
   gaa aaa tgg ctg gaa acg cat ccg atg ccg cag gcg ctg ttc aca acg     1375
   Glu Lys Trp Leu Glu Thr His Pro Met Pro Gln Ala Leu Phe Thr Thr
   230                 235                 240                 245
   tcg ttt gcg ttg ttg caa gga gtg atg gat gtc acg ctg cgt cgc gac     1423
   Ser Phe Ala Leu Leu Gln Gly Val Met Asp Val Thr Leu Arg Arg Asp
                   250                 255                 260
   ggc aaa ctg cct tct gac ctg gca att gcc acc ttt ggc gat aac gaa     1471
   Gly Lys Leu Pro Ser Asp Leu Ala Ile Ala Thr Phe Gly Asp Asn Glu
               265                 270                 275
   ctg ctc gac ttc tta cag tgt ccg gtg ctg gca gtg gct caa cgt cac     1519
   Leu Leu Asp Phe Leu Gln Cys Pro Val Leu Ala Val Ala Gln Arg His
           280                 285                 290
   cgc gat gtc gca gag cgt gtg ctg gag att gtc ctg gca agc ctg gac     1567
   Arg Asp Val Ala Glu Arg Val Leu Glu Ile Val Leu Ala Ser Leu Asp
       295                 300                 305
   gaa ccg cgt aag cca aaa cct ggt tta acg cgc att aaa cgt aat ctc     1615
   Glu Pro Arg Lys Pro Lys Pro Gly Leu Thr Arg Ile Lys Arg Asn Leu
   310                 315                 320                 325
   tat cgc cgc ggc gtg ctc agc cgt agc taa gccgcgaaca aaaatacgcg       1665
   Tyr Arg Arg Gly Val Leu Ser Arg Ser
                   330
   ccaggtgaat ttccctctgg cgcgtagagt acgggactgg acatcaatat gcttaaagta   1725
   aataagacta ttcctgacta ttattgataa atgcttttaa acccgcccgt taattaactc   1785
   accagctgaa attcacaata attaagtgat atcgacagcg cgtttttgca ttattttgtt   1845
   acatgcggcg atgaattgcc gatttaacaa acacttttct ttgcttttgc gcaaacccgc   1905
   tggcatcaag cgccacacag acgtaacaag gactgttaac cggggaagat atgtcctaaa   1965
   atgccgctcg cgtcgcaaac tgacacttta tatttgctgt ggaaaatagt gagtcatttt   2025
   aaaacggtga tgacgatgag ggattttttc ttacagctat tcataacgtt aatttgcttc   2085
   gcacgttgga cgtaaaataa acaacgctga tattagccgt aaacatcggg ttttttacct  2145
   cggtatgcct tgtgactggc t                                             2166
<210> 2
   <211> 334
   <212> PRT
   <213> Escherichia coli
<400> 2
   Met Lys Leu Asp Glu Ile Ala Arg Leu Ala Gly Val Ser Arg Thr Thr
   1 5 10 15
   Ala Ser Tyr Val Ile Asn Gly Lys Ala Lys Gln Tyr Arg Val Ser Asp
   20 25 30
   Lys Thr Val Glu Lys Val Met Ala Val Val Arg Glu His Asn Tyr His
   35 40 45
   Pro Asn Ala Val Ala Ala Gly Leu Arg Ala Gly Arg Thr Arg Ser Ile
   50 55 60
   Gly Leu Val Ile Pro Asp Leu Glu Asn Thr Ser Tyr Thr Arg Ile Ala
   65 70 75 80
   Asn Tyr Leu Glu Arg Gln Ala Arg Gln Arg Gly Tyr Gln Leu Leu Ile
   85 90 95
   Ala Cys Ser Glu Asp Gln Pro Asp Asn Glu Met Arg Cys Ile Glu His
   100 105 110
   Leu Leu Gln Arg Gln Val Asp Ala Ile Ile Val Ser Thr Ser Leu Pro
   115 120 125
   Pro Glu His Pro Phe Tyr Gln Arg Trp Ala Asn Asp Pro Phe Pro Ile
   130 135 140
   Val Ala Leu Asp Arg Ala Leu Asp Arg Glu His Phe Thr Ser Val Val
   145 150 155 160
   Gly Ala Asp Gln Asp Asp Ala Glu Met Leu Ala Glu Glu Leu Arg Lys
   165 170 175
   Phe Pro Ala Glu Thr Val Leu Tyr Leu Gly Ala Leu Pro Glu Leu Ser
   180 185 190
   Val Ser Phe Leu Arg Glu Gln Gly Phe Arg Thr Ala Trp Lys Asp Asp
   195 200 205
   Pro Arg Glu Val His Phe Leu Tyr Ala Asn Ser Tyr Glu Arg Glu Ala
   210 215 220
   Ala Ala Gln Leu Phe Glu Lys Trp Leu Glu Thr His Pro Met Pro Gln
   225 230 235 240
   Ala Leu Phe Thr Thr Ser Phe Ala Leu Leu Gln Gly Val Met Asp Val
   245 250 255
   Thr Leu Arg Arg Asp Gly Lys Leu Pro Ser Asp Leu Ala Ile Ala Thr
   260 265 270
   Phe Gly Asp Asn Glu Leu Leu Asp Phe Leu Gln Cys Pro Val Leu Ala
   275 280 285
   Val Ala Gln Arg His Arg Asp Val Ala Glu Arg Val Leu Glu Ile Val
   290 295 300
   Leu Ala Ser Leu Asp Glu Pro Arg Lys Pro Lys Pro Gly Leu Thr Arg
   305 310 315 320
   Ile Lys Arg Asn Leu Tyr Arg Arg Gly Val Leu Ser Arg Ser
   325 330
<210> 3
   <211> 20
   <212> DNA
   <213> Artificial sequence
<220>
   <221> Primer
   <222> (1)..(20)
   <223> fruR'5'-1
<400> 3
   atgaatcagg cgcgttatcc 20
<210> 4
   <211> 20
   <212> DNA
   <213> Artificial sequence
<220>
   <221> Primer
   <222> (1)..(20)
   <223> fruR'5'-2
<400> 4
   ttgtcgctca cacggtattg 20
<210> 5
   <211> 20
   <212> DNA
   <213> Artificial sequence
<220>
   <221> Primer
   <222> (1)..(20)
   <223> fruR'3'-1
<400> 5
   agcgtgtgct ggagattgtc 20
<210> 6
   <211> 20
   <212> DNA
   <213> Artificial sequence
<220>
   <221> Primer
   <222> (1)..(20)
   <223> fruR'3'-2
<400> 6
   agccagtcac aaggcatacc 20
<210> 7
   <211> 1512
   <212> DNA
   <213> Escherichia coli
<220>
   <221> misc_feature
   <222> (1)..(1512)
   <223> Mutagenic DNA
<220>
   <221> misc_feature
   <222> (1)..(42)
   <223> Technical DNA/ remainder polylinker sequence
<220>
   <221> misc_feature
   <222> (43)..(780)
   <223> Part of the upstream-lying region and part of the 5'-region of the fruR gene
<220>
   <221> misc_feature
   <222> (781)..(837)
   <223> Technical DNA/ remainder polylinker sequence
<220>
   <221> misc_feature
   <222> (838)..(1471)
   <223> Part of the 3'-region of the fruR gene and part of the downstreamlying region
<220>
   <221> misc_feature
   <222> (1472)..(1512)
   <223> Technical DNA/ remainder polylinker sequence
<400> 7
   gatccactag taacggccgc cagtgtgctg gaattcgccc ttatgaatca ggcgcgttat      60
   cccgcgtgat tggccttttt tcccagcgtg gctacaacat tgaaagcctg accgttgcgc     120
   caaccgacga tccgacatta tcgcgtatga ccatccagac cgtgggcgat gaaaaagtac     180
   ttgagcagat cgaaaagcaa ttacacaaac tggtcgatgt cttgcgcgtg agtgagttgg     240
   ggcagggcgc gcatgttgag cgggaaatca tgctggtgaa aattcaggcc agcggttacg     300
   ggcgtgacga agtgaaacgt aatacggaaa tattccgtgg gcaaattatc gatgtcacac     360
   cctcgcttta taccgttcaa ttagcaggca ccagcggtaa gcttgatgca tttttagcat     420
   cgattcgcga tgtggcgaaa attgtggagg ttgctcgctc tggtgtggtc ggactttcgc     480
   gcggcgataa aataatgcgt tgagaatgat ctcaatgcgc aatttacagc ccaacatgtc     540
   acgttgggct ttttttgcga aatcagtggg aacctggaat aaaagcagtt gccgcagtta     600
   attttctgcg cttagatgtt aatgaattta acccatacca gtacaatggc tatggttttt     660
   acattttacg caaggggcaa ttgtgaaact ggatgaaatc gctcggctgg cgggagtgtc     720
   gcggaccact gcaagctatg ttattaacgg caaagcgaag caataccgtg tgagcgacaa     780
   aagggcgaat tctgcagatg gccgccagtg tgatggatat ctgcagaatt cgcccttagc     840
   gtgtgctgga gattgtcctg gcaagcctgg acgaaccgcg taagccaaaa cctggtttaa     900
   cgcgcattaa acgtaatctc tatcgccgcg gcgtgctcag ccgtagctaa gccgcgaaca     960
   aaaatacgcg ccaggtgaat ttccctctgg cgcgtagagt acgggactgg acatcaatat    1020
   gcttaaagta aataagacta ttcctgacta ttattgataa atgcttttaa acccgcccgt    1080
   taattaactc accagctgaa attcacaata attaagtgat atcgacagcg cgtttttgca    1140
   ttattttgtt acatgcggcg atgaattgcc gatttaacaa acacttttct ttgcttttgc    1200
   gcaaacccgc tggcatcaag cgccacacag acgtaacaag gactgttaac cggggaagat    1260
   atgtcctaaa atgccgctcg cgtcgcaaac tgacacttta tatttgctgt ggaaaatagt    1320
   gagtcatttt aaaacggtga tgacgatgag ggattttttc ttacagctat tcataacgtt    1380
   aatttgcttc gcacgttgga cgtaaaataa acaacgctga tattagccgt aaacatcggg    1440
   ttttttacct cggtatgcct tgtgactggc taagggcgaa ttccagcaca ctggcggccg    1500
   ttactagagg gc                                                         1512
<210> 8
   <211> 268
   <212> DNA
   <213> Escherichia coli
<220>
   <221> misc_feature
   <222> (1)..(268)
   <223> Mutagenic DNA
<220>
   <221> misc_feature
   <222> (1)..(3)
   <223> Start codon of the delta fruR allele
<220>
   <221> misc_feature
   <222> (1)..(98)
   <223> 5' -region of the delta fruR allele
<220>
   <221> misc_feature
   <222> (99)..(155)
   <223> Technical DNA/ remainder polylinker sequence
<220>
   <221> misc_feature
<222> (156)..(265)
   <223> 3'-region of the delta fruR allele
<220>
   <221> misc_feature
   <222> (266)..(268)
   <223> Stop codon of the delta fruR allele
<400> 8
   gtgaaactgg atgaaatcgc tcggctggcg ggagtgtcgc ggaccactgc aagctatgtt      60
   attaacggca aagcgaagca ataccgtgtg agcgacaaaa gggcgaattc tgcagatggc     120
   cgccagtgtg atggatatct gcagaattcg cccttagcgt gtgctggaga ttgtcctggc     180
   aagcctggac gaaccgcgta agccaaaacc tggtttaacg cgcattaaac gtaatctcta     240
   tcgccgcggc gtgctcagcc gtagctaa                                        268